(19) Europäisches Patentamt
European Patent Office
Office européen des brevets

(11) Veröffentlichungsnummer : **0 011 255**
**B1**

(12) **EUROPÄISCHE PATENTSCHRIFT**

(45) Veröffentlichungstag der Patentschrift : **26.01.83**

(21) Anmeldenummer : **79104421.7**

(22) Anmeldetag : **09.11.79**

(51) Int. Cl.³ : **C 07 D221/22**, C 07 D491/08, A 61 K 31/435 // (C07D491/08, 317/00, 221/00)

(54) **1,2,3,4-Tetrahydro-6-oxo-2,8a-methano-6H-dibenz(c.e.)azocine, diese enthaltende Arzneimittel und Verfahren zu deren Herstellung.**

(30) Priorität : **15.11.78 DE 2849472**
**07.07.79 DE 2927501**

(43) Veröffentlichungstag der Anmeldung : **28.05.80 Patentblatt 80/11**

(45) Bekanntmachung des Hinweises auf die Patenterteilung : **26.01.83 Patentblatt 83/04**

(84) Benannte Vertragsstaaten : **DE LU**

(56) Entgegenhaltungen :
**CHEMICAL ABSTRACTS, Band 87, Nr. 24, 12. Dezember 1977, Zusammenfassung Nr. 189538s, Seite 320, Columbus, Ohio, US, V. BHASIN et al. : « Thin layer chromatographic studies of some 3, 4 : 6,7-dibenzomorphans — a new class of compounds related to 6,7-benzomorphans ».**
**CHEMICAL ABSTRACTS, Band 86, Nr. 13, 28. März 1977, Zusammenfassung Nr. 90104.q, Seite 572, Columbus, Ohio, US, H. HARA et al. : « Acid-catalyzed reaction of 1-substituted-10-acetoxy-8-chloro-6-methoxy-2-methyl-7-oxodelta 5,6,8,9-hexahydroisoquinolines ».**

(73) Patentinhaber : **GÖDECKE AKTIENGESELLSCHAFT**
**Patentabteilung Postfach 569**
**D-7800 Freiburg (DE)**

(72) Erfinder : **Hartenstein, Johannes, Dr.**
**Fohrenbühl 23**
**D-7801 Stegen-Wittental (DE)**
Erfinder : **Satzinger, Gerhard, Dr.**
**im Mattenbühl 7**
**D-7809 Denzlingen (DE)**
Erfinder : **Bahrmann, Heinrich, Dr.**
**Am Rainhof 29**
**D-7815 Kirchzarten (DE)**
Erfinder : **Ganser, Volker, Dr.**
**Sulzburgerstrasse 22**
**D-7800 Freiburg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**EP 0 011 255 B1**

1,2,3,4-Tetrahydro-6-oxo-2,8a-methano-6H-dibenz(c.e.)azocine, diese enthaltende Arzneimittel und Verfahren zu deren Herstellung

Die Erfindung betrifft ein Verfahren zur Herstellung von Verbindungen, welche ein 1,2,3,4-Tetrahydro-6-oxo-2,8a-methano-6H-dibenz(c.e)azocin-system der allgemeinen Formel I

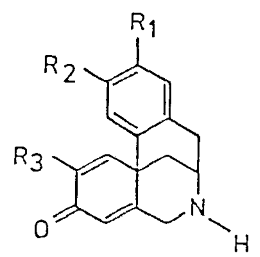

(I)

aufweisen, in welcher

$R_1$ eine Hydroxy- oder eine Alkoxygruppe mit 1-3 Kohlenstoffatomen,

$R_2$ Wasserstoff oder eine Alkoxygruppe mit 1-3 Kohlenstoffatomen,

$R_3$ eine Alkoxygruppe mit 1-3 Kohlenstoffatomen bedeuten, wobei $R_1$ und $R_2$ zusammen auch eine Methylendioxygruppe darstellen können, dadurch gekennzeichnet, daß man ein 3-Benzyl-1,2,3,4-tetrahydroisochinolin-derivat der allgemeinen Formel II

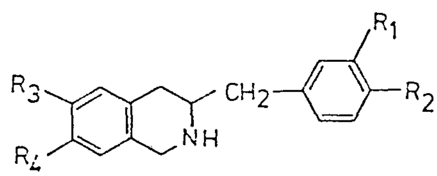

(II)

in welcher die Reste $R_1$, $R_2$ und $R_3$ die obengenannte Bedeutung haben und $R_4$ eine Alkoxygruppe mit 1-3 Kohlenstoffatomen darstellt, deren Wasserstoffatom am Ringstickstoff durch Reste substituiert sein kann, die weder selbst durch die Reaktion verändert werden noch die Reaktion aus sterischen Gründen unterbinden, mittels in organischer Phase löslicher Verbindungen ausreichenden Oxidationspotentials oxidativ cyclisiert. Ein bevorzugtes Oxidationsmittel ist Vanadyltrifluorid.

Die Reaktion ist allgemein durchführbar und führt zu dem bisher nicht bekannten Azocinsystem gemäß Formel I.

Substituenten des Ringstickstoffatoms der Verbindungen gemäß allgemeiner Formel II sind insbesondere Alkylgruppen mit bis zu 8 Kohlenstoffatomen, die geradkettig oder verzweigt sein können, Cycloalkylgruppen mit 3 bis 7 Kohlenstoffatomen oder Cycloalkylalkylgruppen mit 4-8 Kohlenstoffatomen. Es kommen jedoch auch Alkenyl- oder Alkinylgruppen mit bis zu 6 Kohlenstoffatomen, eine 2- oder 3-Furylmethylgruppe oder eine 2- oder 3-Tetrahydrofurylmethylgruppe, welche gegebenenfalls durch 1-3 Methylgruppen substituiert sein können, oder unsubstituierte oder substituierte Phenylniederalkyl-, wie z.B. Benzylgruppen, die durch 1 oder 2 Halogenatome und/oder Hydroxyl-, Methyl-, Trifluormethyl-, Methoxy-, Äthoxy-, Niederalkanoyloxy- oder Methylendioxygruppen substituiert sein können, infrage. Soweit sich derart substituierte Ausgangsprodukte aus Gründen der Reaktivität bzw. Reaktionskinetik nicht unmittelbar umsetzen lassen, führt man den N-Substituente in allgemein bekannter Weise, z.B. mittels der entsprechenden Halogenide erst nach dem Ringschluß ein. Als geradkettige N-Alkylreste kommen die Niederalkylreste Methyl bis Hexyl in Frage. Verzweigte Alkylgruppen sind insbesondere Isopropyl, Isobutyl, und Isopentyl. Cycloalkylreste sind insbesondere Cyclopropyl-, Cyclobutyl-, Cyclopentyl- oder Cyclohexylreste, Cycloalkylalkylreste sind z.B. Cyclopropylmethyl-, Cyclobutylmethyl-, Cyclopentylmethyl- oder Cyclohexyläthylreste. Unter ungesättigten Resten werden insbesondere die Allyl-, Dimethylallyl- und Propargylreste verstanden.

Niederalkanoylreste sind die Acyloxyreste niederer aliphatischer Carbonsäuren wie Formyloxy, Acetoxy, Propionyloxy, Butyryloxy. Diese Gruppen sind nur beispielhaft erwähnt.

Die so erhaltenen Verbindungen der allgemeinen Formel I, welche gewünschtenfalls in der oben angegebenen Weise N-substituiert sein können, stellen wertvolle Zwischenprodukte für die Herstellung von Verbindungen mit Wirkung auf das Zentralnervensystem dar. Teilweise besitzen sie selbst wertvolle pharmakologische Eigenschaften in der genannten Richtung.

Sie stellen insbesondere wertvolle Arzneimittel für die Bekämpfung von Schmerz- und Unruhezuständen dar.

Gegenstand der Erfindung sind weiter Verbindungen der allgemeinen Formel III

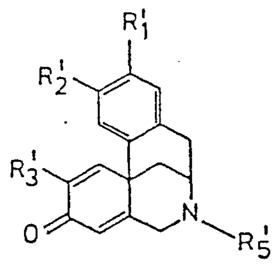

(III)

in welcher
$R'_1$ eine Hydroxy- oder eine Alkoxygruppe mit 1-3 Kohlenstoffatomen,
$R'_2$ Wasserstoff der eine Alkoxygruppe mit 1-3 Kohlenstoffatomen,
$R'_3$ eine Alkoxygruppe mit 1-3 Kohlenstoffatomen und
$R'_5$ Wasserstoff oder eine Alkylgruppe mit 1-3 Kohlenstoffatomen oder eine verzweigte oder geradkettige Alkenylgruppe mit bis zu 5 Kohlenstoffatomen, eine Cycloalkylalkylgruppe mit 4-7 Kohlenstoffatomen oder eine Phenäthylgruppe, deren Phenylrest durch 1 bis 3 Methoxy- oder Äthoxygruppen oder die Methylendioxygruppe substituiert sein kann, bedeuten, wobei $R'_1$ zusammen mit $R'_2$ auch eine Methylendioxygruppe sein kann, sowie deren pharmakologisch verträgliche Salze.

Aus C.A.87, 1895385, sowie C.A.86, 90104q sind bereits 3,4 : 6,7-Dibenzomorphan- bzw. Morphandienonderivate bekannt geworden, über deren pharmakologische Wirksamkeit jedoch nichts ausgesagt ist. Die bekannten Verbindungen unterscheiden sich von den Verbindungen der allgemeinen Formeln I und III vor allem wesentlich durch die Position des zweiten ankondensierten aromatischen Kerns.

Besonders interessant sind Verbindungen der Formel III, in welcher die Reste $R'_1$ und $R'_3$, die gleich oder verschieden sein können, eine Methoxy- oder Äthoxygruppe und der Rest $R'_2$ ein Wasserstoffatom oder eine Methoxy- oder Äthoxygruppe und der Rest $R'_5$ ein Wasserstoffatom oder eine Methylgruppe darstellen,

Als Alkenylgruppen kommen z.B. die Allyl- oder die Butenylgruppe und deren durch Methyl- bzw. Äthylgruppen substituierte Homologe infrage. Cycloalkylalkylgruppen sind etwa die Cyclopropylmethyl-, die Cyclopropyläthyl-, die Cyclopentylmethyl-, die Cyclobutylmethyl- und die Cyclohexylmethylgruppe.

Bevorzugt sind auch diejenigen Verbindungen, in welchen $R'_1$ eine Hydroxylgruppe und/oder $R'_5$ eine Cyclopropylmethylgruppe, eine gegebenenfalls endständig mit bis zu zwei Methylgruppen substituierte Alkenylgruppe oder eine Dimethoxyphenyläthylgruppe bedeutet.

Beansprucht werden auch die pharmakologisch verträglichen Salze der Verbindungen III mit organischen oder anorganischen Säuren. Die Verbindungen III sind besonders ZNS-aktiv und werden deshalb im Rahmen der vorliegenden Erfindung bevorzugt. Gegenstand der Erfindung sind auch Arzneimittel mit einem Gehalt an mindestens einer Verbindung der allgemeinen Formel III und deren pharmakologisch verträglichen Salzen.

Das erfindungsgemäße Verfahren wird durchgeführt, indem man eine Verbindung der allgemeinen Formel II unter Kühlung in Trifluoressigsäure löst und mit einer mindestens äquimolaren Menge, bevorzugt jedoch in einem bis zu 2 1/2 fachen Überschuß Vanadyltrifluorid bei einer Temperatur zwischen − 15 °C und + 30 °C, bevorzugt bei einer Temperatur zwischen − 15 °C und − 5 °C umsetzt und die Verbindungen der allgemeinen Formel I in an sich bekannter Weise isoliert und gewünschtenfalls in ein Salz überführt. Bei der Umsetzung färbt sich das Reaktionsgemisch vorübergehend dunkelblau bis rotviolett. Eine quantitative Umsetzung wird durch einen Überschuß von Vanadyltrifluorid begünstigt. Die Umsetzung ist bei einem Überschuß von 2,5 Mol Vanadyltrifluorid quantitativ. Bei Verwendung von einem geringeren molaren Überschuß oder von äquimolaren Mengen Vanadyltrifluorid bleibt ein Teil des Ausgangsmaterials unumgesetzt.

Nach beendeter Zugabe der Vanadyltrifluorid-Lösung läßt man das Reaktionsgemisch nach 0,5 bis 2 Stunden bei − 10 °C bis 0 °C rühren, zieht die Trifluoressigsäure bei 0 °C bis 20 °C im Vakuum ab und verteilt den Rückstand gegebenenfalls unter Basifizieren, zwischen Wasser und einem organischen Lösungsmittel, wie z.B. Methylenchlorid, Dichloräthylen oder bevorzugt Chloroform.

Aus dem nach üblicher Aufarbeitung der organischen Phase erhaltenen Rohprodukt gewinnt man das Trifluoracetat oder die freie Base durch direkte Kristallisation oder chromatographische Reinigung an einem inerten Trägermaterial wie z.B. Kieselgel.

Die Aufarbeitung kann im einzelnen beispielsweise so durchgeführt werden, daß man nach Entfernung der Trifluoressigsäure das Reaktionsprodukt mit Wasser versetzt, das Gemisch mit Ammoniak basisch stellt und mit Chloroform, Dichloräthylen, Methylenchlorid oder Diäthyläther extrahiert.

Die erfindungsgemäßen Verbindungen der allgemeinen Formel I, deren Wasserstoffatom am Ringstickstoff nicht substituiert ist, können nach an sich bekannten Methoden N-substituiert werden. Sie können z.B. durch Umsetzung mit einem reaktiven Alkylderivat alkyliert werden. Die N-Substitution kann gegebenenfalls auch nach vorausgehender Acylierung durch Reduktion, z.B. mit Lithiumaluminiumhydrid, erreicht werden. In diesem Fall muß die Ketogruppe des Cyclohexadienonringes entweder vorübergehend geschützt werden, beispielsweise durch Überführung in ein Ketal, oder nachträglich

wieder durch Oxidation des entstandenen Aminoalkohols zurückgebildet werden. Die Überführung in ein Salz erfolgt dann durch Umsetzung mit einer äquivalenten Menge einer organischen oder anorganischen Säure. Die Ausgangsprodukte der allgemeinen Formel II sind bekannt, oder können in analoger Weise nach literaturbekannten Methoden hergestellt werden, z.B. nach Arch. Pharm. *297* (1964), 129 ; Ber. *99* (1966) 2873 ; Ber. *73* (1940), 782 ; Eur. J. Med. Chem. *1975,* 10, 19.

Oxidative Cyclisierungen von 3-Benzyl-1,2,3,4-tetrahydroisochinolinen sind bisher noch nicht in der Literatur beschrieben worden. Die mit hoher Regioselektivität verlaufende Reaktion ist für den Fachmann nicht vorhersehbar und überraschend. Durch das erfindungsgemäße Verfahren wird ein Zugang zu einer neuer Stoffklasse geschaffen.

Die Überführung der freien Basen der allgemeinen Formel III in deren pharmakologisch verträgliche Salze erfolgt durch Neutralisation mit einer entsprechend anwendbaren organischen oder anorganischen Säure, wie z.B. Salzsäure, Schwefelsäure, Phosphorsäure, Bromwasserstoffsäure, Essigsäure, Fumarsäure, Oxalsäure, Milchsäure, Zitronensäure, Äpfelsäure, Salicylsäure, Malonsäure, Maleinsäure, Bernsteinsäure oder Ascorbinsäure. Zur Herstellung von Arzneimitteln werden die Wirkstoffe mit üblichen Zusätzen und flüssigen oder festen Trägerstoffen verarbeitet. Die Verbindungen der Formel III können in weiten Dosierungsgrenzen in flüssiger oder fester Form oral oder parenteral appliziert werden.

Übliche Zusätze für flüssige Formen sind z.B. Tartrat- und Citrat-Puffer, Äthanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol) ; für orale Applikation geeignete Zubereitungen können gewünschtenfalls Geschmacks- und Süßstoffe enthalten.

Die Einzeldosis der erfindungsgemäßen Verbindungen dürfte je nach Indikation im Bereich von 25-100 mg liegen.

Die Wirkung der Verbindungen der Beispiele 1 bis 4 wird im folgenden Versuchsbericht beschrieben :

## Versuchsbericht

(±)-1,2,3,4-Tetrahydro-7,10,11-trimethoxy-3-methyl-6-oxo-2,8a-methano-6H-dibenz(c.e.)azocin, (±)-1,2,3,4-Tetrahydro-7,10,11-trimethoxy-6-oxo-2,8a-methano-6H-dibenz(c.e.)azocin, (±)-7,10,11-Triäthoxy-1,2,3,4-tetrahydro-3-methyl-6-oxo-2,8a-methano-6H)-dibenz(c.e.)azocin und (±)-1,2,3,4-Tetrahydro-7,11-dimethoxy-6-oxo-2,8a-methano-6H-dibenz(c.e.)azocin (Beispiele 1 bis 4) wurden auf analgetische Wirkung untersucht.

Die $LD_{50}$-Werte bei der Maus, nach intragastraler Verabreichung der Substanzen in Wasser, lagen bei 400 mg/kg und darüber.

Bei der Untersuchung von Mäusen im Heizplattentest (J. Pharmacol. Exper. Therap. *80,* 300 (1944)) wurde bei subcutaner Verabreichung von 30-50 mg/kg eine deutliche analgetische Wirkung festgestellt. Bereits ab 50 mg/kg wurde zusätzlich eine starke sedierende Wirkung festgestellt.

Im Phenyl-p-chinon-Test (Biol. Med. *95,* 729 (1957)) war die analgetische Wirkung an der Maus nach subcutaner und intragastraler Application schwächer, aber immer noch deutlich ausgeprägt.

Die im Heizplattentest festgestellte sedierende Wirkung konnte im Motilitätstest (Screening methods in Pharmacology S. 78, Acad. Press 1965, New York — London) an der Maus bestätigt werden.

Die Verbindungen der Beispiele 1 bis 4 stellen somit Analgetika mit sedativen Eigenschaften dar.

Die folgenden Beispiele dienen der Erläuterung der vorliegenden Erfindung :

## Beispiel 1

(±)-1,2,3,4-Tetrahydro-7,10,11-trimethoxy-3-methyl-6-oxo-2,8a-methano-6H-dibenz(c.e.)azocin

5,63 g (15,74 mmol) 2-Methyl-6,7-dimethoxy-3-veratryl-1,2,3,4-tetrahydroisochinolin, (Arch. Pharm. *297* (1964) 129), werden unter Kühlen mit Eiswasser im 60 ml Trifluoressigsäure gelöst und unter Feuchtigkeitsausschluß in einer Stickstoffschutzatmosphäre bei − 15 °C bis- − 10 °C mit einer Lösung von 4,20 g (2,5 Äquivalenten) Vanadyltrifluorid in 200 ml Trifluoressigsäure während 5 Minuten tropfenweise versetzt. Man läßt noch 1 Stunde bei − 15 °C bis − 10 °C nachrühren und destilliert dann die Trifluoressigsäure bei Raumtemperatur/100 Torr ab. Der Rückstand wird mit Wasser versetzt und mit Chloroform extrahiert. Die vereinigten Chloroformextrakte wäscht man mit halbkonzentriertem wäßrigem Ammoniak. Nach Trocknen und Eindampfen und Kristallisation des Rückstands aus Methanol/Äther erhält man 4,55 g (85 %) (±)-1,2,3,4-Tetrahydro-7,10,11-trimethoxy-3-methyl-6-oxo-2,8a-methano-6H-dibenz(c.e.)azocin vom Schmp. 168,5-170 °C.

MS : $M^+$ 341 ; IR (KBr) : 1 660, 1 633, 1 610 $cm^{-1}$

NMR ($CDCl_3$, δ) 2,54 ($N-CH_3$), 3,72, 3,77, 3,88 ($3xOCH_3$) 5,94, 6,15, 6,77 (4x1H, s).

Durch Behandeln mit methanolischer wäßriger Salzsäure und Kristallisation aus Methanol/Äther erhält man (±)-1,2,3,4-Tetrahydro-7,10,11-trimethoxy-3-methyl-6-oxo-2,8a-methano-6H-dibenz(c.e.)azo-

cin. Hydrochlorid (Zers. 250 °C).

$C_{20}H_{23}NO_4 \cdot HCl$

Ber. : C 63,57 H 6,40 Cl 9,38 N 3,71 %

Gef. : C 63,37 H 6,32 Cl 9,56 N 3,60 %

UV : $\lambda_{max}$ (MeOH) 242 nm (18 400), 283 nm (5 400).

## Beispiel 2

(±)-1,2,3,4-Tetrahydro-7,10,11-trimethoxy-6-oxo-2,8a-methano-6H-dibenz(c.e.)azocin

15,2 g (40 mmol), 6,7-Dimethoxy-3-veratryl-1,2,3,4-tetrahydroisochinolin · Hydrochlorid, (Ber. *73,* S. 782 (1940)) werden mit wäßrigem Ammoniak in die freie Base übergeführt. Nach Extraktion mit Chloroform und Trocknen des Extrakts wird im Vakuum eingedampft, und der Rückstand bei 0 °C in 110 ml Trifluoressigsäure aufgenommen. Zu dieser Lösung läßt man bei – 15 °C unter Feuchtigkeitsausschluß in einer Schutzgasatmosphäre (Stickstoff) eine Suspension von 12 g (100 mmol) Vanadyltrifluorid in 370 ml Trifluoressigsäure innerhalb von 15 Minuten unter Rühren zutropfen. Danach wird das dunkelgefärbte Reaktionsgemisch noch 45 Minuten bei – 10 °C gerührt und anschließend bei 15-20 °C/100 Torr während 1 Stunde eingedampft. Man verteilt den Rückstand zwischen Wasser und Chloroform. Die Chloroform-Phase wird dann mit halbkonzentriertem wäßrigem Ammoniak behandelt. Die organische Phase wird nach Trocknen im Vakuum eingedampft. Der hinterbleibende gelbliche Schaum kristallisiert aus Methanol/Äther in Form farbloser Kristalle. Man erhält 8,80 g (67,2 % d. Th.) (±)-1,2,3,4-Tetrahydro-7,10,11-trimethoxy-6-oxo-2,8a-methano-6H-dibenz(c.e.)azocin von Schmp. 186-187 °C.

MS : $M^+$ 327

IR (KBr) : 1 655, 1 633, 1 610 $cm^{-1}$

NMR ($CDCl_3$, δ) 3,70, 3,73, 3,89, ($3xOCH_3$), 5,93, 6,10, 6,50, 6,74 (4x1H, s).

Behandeln der Base mit methanolischer Salzsäure liefert das kristalline Hydrochlorid vom Schmp. 222-226 °C.

$C_{19}H_{21}NO_4 \cdot HCl$

Ber. : C 62,72 H 6,09 Cl 9,74 N 3,85 %

Gef. : C 62,49 H 6,06 Cl 9,95 N 3,81 %

UV : $\lambda_{max}$ (MeOH) 238 nm (17 200), 275 (6 000).

## Beispiel 3

(±)-7,10,11-Triäthoxy-1,2,3,4-tetrahydro-3-methyl-6-oxo-2,8a-methano-6H-dibenz(c.e.)azocin

8,9 g (21,5 mmol) 6,7-Diäthoxy-3-(3,4-diäthoxybenzyl)-2-methyl-1,2,3,4-tetrahydroisochinolin (Ber. *99* (1966), S. 2873) werden bei 0 °C in 80 ml Trifluoressigsäure gelöst. Zu dieser Lösung läßt man bei – 15 °C unter Rühren und Feuchtigkeitsausschluß (Stickstoffatmosphäre) eine Lösung von 6,6 g (2,5 Äquivalente) Vanadyltrifluorid in 250 ml Trifluoressigsäure zutropfen. Das Reaktionsgemisch färbt sich dabei tiefrotviolett. Man läßt noch 1 Stunde bei – 10 °C nachrühren und arbeitet dann, wie in Beispiel 1 beschrieben, auf. Kristallisation aus Methanol ergibt 5,92 g (72 %) (±)-7,10,11-Triäthoxy-1,2,3,4-tetrahydro-3-methyl-6-oxo-2,8a-methano-6H-dibenz(c.e.)azocin vom Schmp. 158-159 °C.

MS : $M^+$ 383

IR (KBr) : 1 668, 1 644, 1 618 $cm^{-1}$,

NMR ($CDCl_3$, δ) 1,4 und 4,0 (m, $3xOCH_2CH_3$), 2,52 ($N-CH_3$), 5,89, 6,10, 6,50, 6,72, (4x1H, s).

Behandeln mit methanolischer Salzsäure und Kristallisation aus Methanol/Äther liefern das Hydrochlorid vom Schmp. 212-215 °C.

$C_{23}H_{29}NO_4 \cdot HCl$

Ber. : C 65,78 H 7,20 Cl 8,44 N 3,34 %

Gef. : C 65,56 H 7,19 Cl 8,42 N 3,26 %

UV : $\lambda_{max}$ (Methanol) 238 nm (18 100), 275 nm (5 900).

## Beispiel 4

(±)-3-[2-(3,4-Dimethoxyphenyl)äthyl]-1,2,3,4-tetrahydro-7,10,11-trimethoxy-6-oxo-2,8a-methano-6H-dibenz(c.e.)azocin

2 g (6,1 mmol) (±)-1,2,3,4-Tetrahydro-7,10,11-trimethoxy-6-oxo-2,8a-methano-6H-dibenz(c.e.)azocin und 1,65 g (6,7 mmol) 2-(3,4-Dimethoxyphenyl)-äthylbromid werden in 30 ml Dimethylformamid gelöst und mit einem Körnchen Kaliumjodid sowie 3,5 g trockenem Kaliumcarbonat versetzt. Man erhitzt das Gemisch unter Stickstoff 3 Stunden auf 70 °C und 3 Stunden auf 100 °C. Nach Abkühlen verteilt man zwischen Wasser und Methylenchlorid. Das nach Trocknen der organischen Phase (Natriumsulfat) und Abziehen des Lösungsmittels anfallende Rohprodukt wird an Kieselgel mit Chloroform als Eluens

chromatographiert. Durch Behandeln der Base mit Bromwasserstoff und Kristallisation aus Methanol/Essigester erhält man (±)-3-(2-(3,4-Dimethoxyphenyl)äthyl)-1,2,3,4-tetrahydro-7,10,11-trimethoxy-6-oxo-2,8a-methano-6H-dibenz(c.e.)azocin in Form des farblosen Hydrobromids vom Schmp. 206-8 °C.

$C_{29}H_{23}NO_6 \cdot HBr$

Ber. : C 60,84 H 5,99 Br 13,96 N 2,45 %

Gef. : C 60,87 H 5,82 Br 13,85 N 2,40 %

IR (KBr) : 1 662, 1 645, 1 615 $cm^{-1}$

NMR ($CDCl_3$, δ) : 3,75, 3,80, 3,86, 3,90 (15 H, $5xOCH_3$), 5,97, 6,50, 6,37, (3x1H, s), 6,82 (3H, m), 7,00 (1 H, s)

UV : $\lambda_{max}$ (Äthanol) 234 nm (25 200), 279 nm (8 600).

## Beispiel 5

(±)-1,2,3,4-Tetrahydro-7-methoxy-10,11-methylendioxy-6-oxo-2,8a-methano-6H-dibenz(c.e.)azocin

5,3 g (14,6 mmol) (±)-3-(3,4-Methylendioxybenzyl)-6,7-dimethoxy-1,2,3,4-tetrahydroisochinolin-Hydrochlorid werden in üblicher Weise durch Behandeln mit Ammoniak in die freie Base übergeführt und bei 0 °C in 20 ml Trifluoressigsäure gelöst und unter Feuchtigkeitsausschluß in einer Stickstoffatmosphäre bei – 10 °C mit einer Lösung von 4,5 g (36,5 mmol) Vanadyltrifluorid in 100 ml Trifluoressigsäure während 10 Minuten unter Rühren tropfenweise versetzt. Man läßt das dunkelblau gefärbte Gemisch jeweils 1 Stunde bei – 10 °C, 0 °C und 22 °C rühren und destilliert dann die Trifluoressigsäure bei Raumtemperatur und 100 Torr ab. Der Rückstand wird mit Wasser versetzt und mit Chloroform extrahiert. Die vereinigten Chloroformextrakte wäscht man mit halbkonzentriertem wäßrigem Ammoniak. Nach Trocknen und Eindampfen und Kristallisation aus Methanol erhält man (±)-1,2,3,4-Tetrahydro-7-methoxy-10,11-methylendioxy-6-oxo-2,8a-methano-6H-dibenz(c.e.)azocin als Base vom Schmp. 199-203 °C.

MS : $M^+$ 311

IR (KBr) : 3 010, 1 658, 1 640, 1 610 $cm^{-1}$

NMR (DMSO-d6 ; δ) : 3,67 ($OCH_3$), 5,87 (3H, $OCH_2O$ = CH—), 6,05 (1H, s), 6,45, 6,80 (2x1H, s).

Durch Behandeln mit äthanolischer Salzsäure und Kristallisation aus Äthanol/Äther erhält man das Hydrochlorid des (±)-1,2,3,4-Tetrahydro-7-methoxy-10,11-methylendioxy-6-oxo-2,8a-methano-6H-dibenz(c.e.)azocins vom Schmp. 235-8 °C.

$C_{18}H_{17}NO_4 \cdot HCl \cdot 0{,}5\ H_2O$

Ber. : C 60,58 H 5,36 Cl 9,94 N 3,92 %

Gef. : C 60,95 H 5,61 Cl 9,75 N 3,78 %

UV : $\lambda_{max}$ (Wasser) 241 nm (14 100), 288 nm (5 400).

## Beispiel 6

(±)-1,2,3,4-Tetrahydro-11-hydroxy-7-methoxy-6-oxo-2,8a-methano-6H-dibenz(c.e.)azocin

0,9 g (3 mmol) (±)-3-(3-Hydroxybenzyl-6,7-dimethoxy-1,2,3,4-tetrahydroisochinolin werden bei 0 °C in einem Gemisch von 10 ml absolutem Methylenchlorid und 10 ml Trifluoressigsäure gelöst. Zu dieser Lösung läßt man in einer Schutzgasatmosphäre bei – 10 °C innerhalb von 5 Minuten eine Lösung von 0,34 ml (3,6 mmol) Vanadylchlorid in 10 ml absolutem Methylenchlorid zutropfen. Man läßt das Gemisch 1 Stunde bei – 10 °C rühren und fügt dann nochmals eine Lösung von 0,17 ml Vanadylchlorid in 5 ml absolutem Methylenchlorid zu. Nach Rühren für 30 Minuten bei – 10 °C und 90 Minuten bei 0 °C wird bei 20 °C in Vakuum konzentriert, der Rückstand mit 20 ml Wasser versetzt und mit Chloroform/Äthanol (8 : 2) erschöpfend extrahiert. Die Aufarbeitung gemäß Beispiel 1 liefert 0,74 g (±)-1,2,3,4-Tetrahydro-11-hydroxy-7-methoxy-6-oxo-2,8a-methano-6H-dibenz-(c.e.)azocin in Form des kristallinen Trifluoracetats. Durch Behandeln mit Ammoniakwasser läßt sich daraus die freie Base vom Schmp. 265 °C (Zers.) gewinnen.

MS : $M^+$ 283

IR (KBr) : 3 285, 1 660, 1 638, 1 615 $cm^{-1}$

NMR (DMSO-$d_6$, δ) : 3,67 ($OCH_3$), 5,95, 6,07 (2x1H, s), 6,67 (3H, m)

$C_{17}H_{17}No_3 \cdot 1/4H_2O$

Ber. : C 71,17 H 6,01 N 4,76 %

Gef. : C 70,93 H 6,13 N 4,86 %.

## Beispiel 7

(±)-3-Allyl-1,2,3,4-tetrahydro-7,10,11-trimethoxy-6-oxo-2,8a-methano-6H-dibenz(c.e.)azocin

4 g (12,2 mmol) (±)-1,2,3,4-Tetrahydro-7,10,11-trimethoxy-6-oxo-2,8a-methano-6H-dibenz(c.e.)azocin werden in 160 ml absolutem Äthanol gelöst und mit 1,72 g (14,2 mmol) Allylbromid versetzt. Nach Zugabe von 2,5 g Natriumhydrogencarbonat läßt man das Gemisch 2 Stunden in einer Stickstoffatmosphäre unter Rückfluß kochen. Danach wird das Lösungsmittel im Vakuum abgezogen. Verteilung des Rückstands zwischen Ammoniakwasser und Methylenchlorid und übliche Aufarbeitung der organischen Phase liefern das rohe N-Allylierungsprodukt, das in Äthanol nach Zugabe von Chlorwasserstoff als Hydrochlorid kristallisiert. Man erhält 3,81 g (77 % d. Th.) (±)-3-Allyl-1,2,3,4-tetrahydro-7,10,11-trimethoxy-6-oxo-2,8a-methano-6H-dibenz-(c.e.)azocin, Schmp. 203-4 °C.

$C_{22}H_{25}NO_4 \cdot HCl$

Ber.: C 65,42 H 6,49 Cl 8,78 N 3,47 %

Gef.: C 65,51 H 6,40 Cl 8,75 N 3,41 %

IR (KBr): 1 663, 1 645, 1 615 $cm^{-1}$

NMR ($CDCl_3$, δ): 3,73, 3,80, 3,87 ($3xOCH_3$), 5,35-5,65 (3H, m), 5,96, 6,33, 6,48, 6,78 (4x1H, s)

UV: $\lambda_{max}$ (Äthanol) 240 nm (16 800), 280 nm (5 200).

Das verwendete Ausgangsprodukt erhält man gemäß Beispiel 2.

## Beispiel 8

(±)-3-[2-(3,4-Dimethoxyphenyl)äthyl]-1,2,3,4-tetrahydro-7,10,11-trimethoxy-6-oxo-2,8a-methano-6H-dibenz(c.e.)azocin

2 g (6,1 mmol) (±)-1,2,3,4-Tetrahydro-7,10,11-trimethoxy-6-oxo-2,8a-methano-6H-dibenz(c.e.)azocin und 1,65 g (6,7 mmol) 2-(3,4-Dimethoxyphenyl)-äthylbromid werden in 30 ml Dimethylformamid gelöst und mit einem Körnchen Kaliumjodid sowie 3,5 g trockenem Kaliumcarbonat versetzt. Man erhitzt das Gemisch unter Stickstoff 3 Stunden auf 70 °C und 3 Stunden auf 100 °C. Nach Abkühlen verteilt man zwischen Wasser und Methylenchlorid. Das nach Trocknen der organischen Phase (Natriumsulfat) und Abziehen des Lösungsmittels anfallende Rohprodukt wird an Kieselgel mit Chloroform als Eluens chromatographiert. Durch Behandeln der Base mit Bromwasserstoff und Kristallisation aus Methanol/Essigester erhält man (±)-3-(2-(3,4-Dimethoxyphenyl)äthyl)-1,2,3,4-tetrahydro-7,10,11-trimethoxy-6-oxo-2,8a-methano-6H-dibenz(c.e.)azocin in Form des farblosen Hydrobromids vom Schmp. 206-8 °C.

$C_{29}H_{23}NO_6 \cdot HBr$

Ber.: C 60,84 H 5,99 Br 13,96 N 2,45 %

Gef.: C 60,87 H 5,82 Br 13,85 N 2,40 %

IR (KBr): 1 662, 1 645, 1 615 $cm^{-1}$

NMR ($CDCl_3$, δ): 3,75, 3,80, 3,86, 3,90 (15H, $5xOCH_3$), 5,97, 6,50, 6,37, (3x1H, s), 6,82 (3H, m), 7,00 (1H, s)

UV: $\lambda_{max}$ (Äthanol) 234 nm (25 200), 279 nm (8 600).

## Beispiel 9

(±)-3-Cyclopropylmethyl-1,2,3,4-tetrahydro-7,10,11-trimethoxy-6-oxo-2,8a-methano-6H-dibenz(c.e.)azocin

4 g (12,2 mmol) (±)-1,2,3,4-Tetrahydro-7,10,11-trimethoxy-6-oxo-2,8a-methano-6H-dibenz(c.e.)azocin und 1,21 g (13,4 mmol) Cyclopropylmethylchlorid werden in 50 ml Dimethylformamid gelöst. Nach Zugabe von 7 g trockenem Kaliumcarbonat und einem Körnchen Kaliumjodid erhitzt man das Gemisch 3 Stunden auf 70 °C, danach 3 Stunden auf 100 °C. Aufarbeitung wie in Beispiel 8 beschrieben und Chromatographie an Kieselgel mit Chloroform/Methanol (95 : 5) als Eluens liefern die Base als gelben Sirup. Durch Behandeln mit Bromwasserstoff und Kristallisation aus Methanol/Äther erhält man (±)-3-Cyclopropylmethyl-1,2,3,4-tetrahydro-7,10,11-trimethoxy-6-oxo-2,8a-methano-6H-dibenz(c.e.)azocin in Form des gelbstichigen Hydrobromids. Zersetzungspunkt ≈ 335 °C.

MS (Base): $M^+$ 381

$C_{23}H_{27}NO_4 \cdot HBr$

Ber.: C 59,74 H 6,10 Br 17,28 N 3,03 %

Gef.: C 59,56 H 6,11 Br 17,15 N 2,95 %

IR (KBr): 1 670, 1 650, 1 620 $cm^{-1}$

NMR (DMSO-$d_6$, δ): 0,4-0,8 (4H, m), 3,66, 3,73, 3,80 ($3xOCH_3$), 6,17, 6,40, 6,55, 6,97 (4x1H, s).

## Beispiel 10

(+)-1,2,3,4-Tetrahydro-7,11-dimethoxy-3-(3-methyl-2-butenyl)-6-oxo-2,8a-methano-6H-dibenz(c.e.)azocin

1 g (3,36 mmol) (±)-1,2,3,4-Tetrahydro-7,11-dimethoxy-6-oxo 2,8a-methano-6H-dibenz(c.e.)azocin wird in 15 ml absolutem Dimethylformamid gelöst und bei Raumtemperatur mit 0,35 g (3,36 mmol) 1-Chlor-3-methyl-2-buten, 1,6 g trockenem Kaliumcarbonat und 50 mg Kaliumjodid versetzt. Unter Rühren in einer Stickstoffatmosphäre wird das Gemisch 2,5 Stunden auf 80 °C erhitzt und danach bei 40 °C im Vakuum eingedampft. Den Rückstand verteilt man zwischen Methylenchlorid und Wasser. Aufarbeitung der organischen Phase in üblicher Weise liefert ein rotbraunes Öl, das durch Chromatographie an

Kieselgel und/oder Kristallisation Äthanol gereinigt wird. Man erhält 0,8 g (65 % d. Th.) (±)-1,2,3,4-Tetrahydro-7,11-dimethoxy-3-(3-methyl-2-butenyl)-6-oxo-2,8a-methano-6H-dibenz(c.e.)azocin in Form farbloser Kristalle vom Schmp. 182-4 °C.

MS : $M^+$ 365
$C_{23}H_{27}NO_3$
Ber. : C 75,58 H 7,75 N 3,83 %
Gef. : C 75,76 H 7,53 N 3,59 %
IR (KBr) : 1 655, 1 635, 1 610, 1 600 $cm^{-1}$
NMR ($CDCl_3$, δ) : 1,69, 1,76 (2x3H, $2xCH_3$), 3,76, 3,79 ($2xOCH_3$), 5,28 (1H, m), 5,90, 6,06 (2x1H, s) 6,53-7,04 (3H, m).

Die als Ausgangsprodukt verwendete Verbindung erhält man gemäß Beispiel 4.

## Beispiel 11

(±)-3-Allyl-1,2,3,4-tetrahydro-7,11-dimethoxy-6-oxo-2,8a-methano-6H-dibenz(c.e.)azocin

3,06 g (7,4 mmol) (±)-1,2,3,4-Tetrahydro-7,11-dimethoxy-6-oxo-2,8a-methano-6H-dibenz(c.e.)azocin · Hydrochlorid werden mit Ammoniakwasser in die Base übergeführt. Nach Extraktion mit Methylenchlorid, Trocknen des Extraktes über Natriumsulfat und Abziehen des Lösungsmittels wird der Rückstand in 100 ml absolutem Äthanol gelöst und mit 0,90 ml (10,4 mmol) Allylbromid versetzt. Man gibt 1,8 g Natriumhydrogencarbonat dazu und erhitzt das Gemisch unter Rühren und Stickstoff 90 Minuten zum Rückfluß. Die Aufarbeitung erfolgt wie in Beispiel 7 beschrieben. Nach Kristallisation aus Äthanol/Äther erhält man (±)-3-Allyl-1,2,3,4-tetrahydro-7,11-dimethoxy-6-oxo-2,8a-methano-6H-dibenz(c.e.)azocin in Form des farblosen Hydrochlorids vom Schmp. 215 °C.

$C_{21}H_{23}NO_3 \cdot HCl$
Ber. : C 67,46 H 6,47 Cl 9,49 N 3,75 %
Gef. : C 67,92 H 6,54 Cl 9,29 N 3,64 %
MS : (Base) 337
IR (KBr) : 1 660, 1 640, 1 610, 1 600 $cm^{-1}$
NMR ($CDCl_3$, δ) : 3,76 ($2xOCH_3$), 5,00-5,40 (3H, m), 5,88, 6,03 (2x1H, s), 6,73 (3H, m)
UV : $\lambda_{max}$ 234 nm (20 900), 275 nm (5 600).

## Beispiel 12

(±)-3-[2-(3,4-Dimethoxyphenyl)äthyl]-1,2,3,4-tetrahydro-7,11-dimethoxy-6-oxo-2,8a-methano-6H-dibenz-(c.e.)azocin

2,02 g (6,05 mmol) (±)-1,2,3,4-Tetrahydro-7,11-dimethoxy-6-oxo-2,8a-methano-6H-dibenz(c.e.)azocin · Hydrochlorid werden in 35 ml absolutem Dimethylformamid bei Raumtemperatur gelöst, mit 1,63 g (6,66 mmol)2-(3,4-Dimethoxyphenyl)-äthylbromid, 3,50 g trockenem Kaliumcarbonat und 0,25 g Kaliumjodid versetzt. Man erhitzt das Gemisch unter Rühren in einer Stickstoffatmosphäre 20 Stunden auf 80 °C, dampft dann im Vakuum bei 40 °C ein und verteilt den Rückstand zwischen Wasser und Methylenchlorid. Durch Aufarbeitung der organischen Phase und chromatographische Reinigung des dabei anfallenden Rohprodukts an Kieselgel (Eluens : Chloroform/Methanol 98 : 2) erhält man (±)-3-(2-(3,4-Dimethoxyphenyl)äthyl)-1,2,3,4-tetrahydro-7,11-dimethoxy-6-oxo-2,8a-methano-6H-dibenz(c.e.)azocin als Base, welche in Äthanol durch Zugabe der äquivalenten Menge Chlorwasserstoff in das Hydrochlorid vom Schmp. 201-3 °C übergeführt wird.

MS(Base) : $M^+$ 283
IR (KBr) : 1 675, 1 655, 1 625, 1 615
$C_{28}H_{31}NO_5 \cdot HCl \cdot 0,5H_2O$
Ber. : C 66,33 H 6,56 Cl 7,00 N 2,76 %
Gef. : C 66,56 H 6,50 Cl 7,27 N 2,72 %
NMR (DMSO-$d_6$) : 3,70, 3,77, (12 H, $4xOCH_3$), 6,20, 6,31, (2x1H, s), 6,96 (6H, m).

**Ansprüche**

1. Verbindungen der allgemeinen Formel III

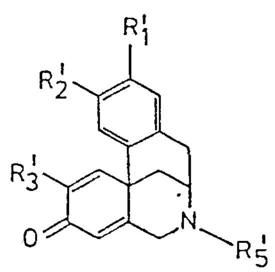

(III)

in welcher

$R'_1$ eine Hydroxy- oder eine Alkoxygruppe mit 1-3 Kohlenstoffatomen,

$R'_2$ Wasserstoff oder eine Alkoxygruppe mit 1-3 Kohlenstoffatomen,

$R'_3$ eine Alkoxygruppe mit 1-3 Kohlenstoffatomen und

$R'_5$ Wasserstoff oder eine Alkylgruppe mit 1-3 Kohlenstoffatomen oder eine verzweigte oder geradkettige Alkenylgruppe mit bis zu 5 Kohlenstoffatomen, eine Cycloalkylalkylgruppe mit 4-7 Kohlenstoffatomen oder eine Phenäthylgruppe, deren Phenylrest durch 1 bis 3 Methoxy- oder Äthoxygruppen oder die Methylendioxygruppe substituiert sein kann, bedeuten, wobei $R'_1$ zusammen mit $R'_2$ auch eine Methylendioxygruppe sein kann, sowie deren pharmakologisch verträgliche Salze.

2. Arzneimittel mit ZNS-Wirkung, gekennzeichnet durch einen Gehalt an mindestens einer Verbindung gemäß Anspruch 1.

3. Verfahren zur Herstellung von Verbindungen der allgemeinen Formel I

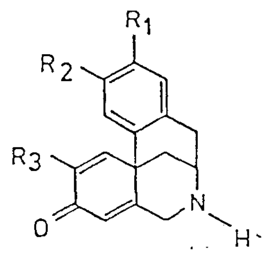

(I)

in welcher

$R_1$ eine Hydroxy- oder eine Alkoxygruppe mit 1-3 Kohlenstoffatomen,

$R_2$ Wasserstoff oder eine Alkoxygruppe mit 1-3 Kohlenstoffatomen,

$R_3$ eine Alkoxygruppe mit 1-3 Kohlenstoffatomen bedeuten, wobei $R_1$ und $R_2$ zusammen auch eine Methylendioxygruppe darstellen können, sowie von deren N-substituierten Derivaten, dadurch gekennzeichnet, daß man ein 3-Benzyl-1,2,3,4-tetrahydro-isochinolinderivat der allgemeinen Formel II

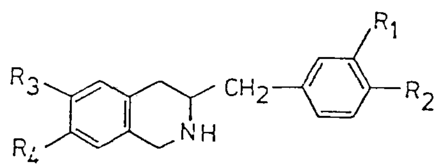

(II)

in welcher die Reste $R_1$, $R_2$ und $R_3$ die obengenannte Bedeutung haben und $R_4$ eine Alkoxygruppe mit 1-3 Kohlenstoffatomen darstellt, wobei deren Wasserstoffatom am Ringstickstoff durch Reste substituiert sein kann, die weder selbst durch die Reaktion verändert werden noch die Reaktion aus sterischen Gründen unterbinden oxidativ mittels in organischer Phase löslicher Verbindungen ausreichenden Oxidationspotentials cyclisiert, und gegebenenfalls anschließend in an sich bekannter Weise N-substituiert und in ein Salz überführt.

4. Verfahren gemäß Anspruch 3, dadurch gekennzeichnet, daß man als Oxidationsmittel Vanadyltrifluorid in Trifluoressigsäure anwendet.

5. Verfahren gemäß Anspruch 3 bis 4, dadurch gekennzeichnet, daß man Vanadyltrifluorid in 2,5 fachem molarem Überschuß verwendet.

6. Verfahren gemäß Anspruch 3 bis 5, dadurch gekennzeichnet, daß man bei einer Temperatur zwischen − 15 °C und − 5 °C umsetzt.

**Claims**

1. Compounds having the following general formula III,

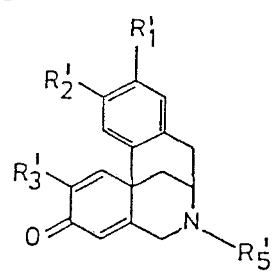

(III)

wherein

$R'_1$ is a hydroxy- or alkoxy group from 1-3 carbon atoms,

$R'_2$ is a hydrogen atom or an alkoxy group from 1-3 carbon atoms,

$R'_3$ is an alkoxy group from 1-3 carbon atoms,

R'$_5$ is a hydrogen atom or an alkyl group from 1-3 carbon atoms or a branched or straight alkenyl group having up to 5 carbon atoms, a cycloalkyl-alkyl group from 4-7 carbon atoms or a phenethyl group, the phenyl radical of which may be substituted by 1 to 3 methoxy or ethoxy groups or the methylenedioxy group and wherein R'$_1$ together with R'$_2$ may also represent a methylenedioxy group and their pharmacologically compatible salts.

2. Pharmaceutical composition with CNS-activity, characterized by containing at least one compound according to claim 1.

3. Process for the manufacture of compounds of the general formula I and of their N-substituted derivatives

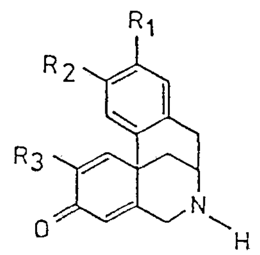

(I)

wherein

$R_1$ is a hydroxy or an alkoxy group from 1-3 carbon atoms,

$R_2$ is hydrogen or an alkoxy group from 1-3 carbon atoms,

$R_3$ is an alkoxy group from 1-3 carbon atoms, wherein $R_1$ and $R_2$ taken together may also represent a methylenedioxy group, characterized by oxidatively cyclizing a 3-benzyl-1,2,3,4-tetrahydroisoquinoline-derivative of the general formula II

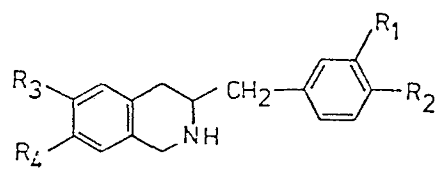

(II)

wherein the radicals $R_1$, $R_2$ and $R_3$ have the above meaning and wherein $R_4$ is representing an alkoxy group from 1-3 carbon atoms and wherein the hydrogen atom which is attached to the ring-nitrogen atom may be substituted by radicals which are not altered by the reaction and do not suppress the reaction by sterical reasons with compounds which are soluble in the present organic phase and which have a sufficient oxidizing potential whereafter the products obtained are optionally N-substituted and converted into a salt.

4. Process according to claim 3, characterized by using vanadyltrifluoride in trifluoro-acetic acid as oxidizing agent.

5. Process according to claims 3 to 4 characterized by using vanadyl-trifluoride in a 2.5 fold molar excess.

6. Process according to process 3 to 5 characterized by carrying out the reaction with a temperature between − 15 °C and − 5 °C.

**Revendications**

1. Composés présentant la formule générale III suivante

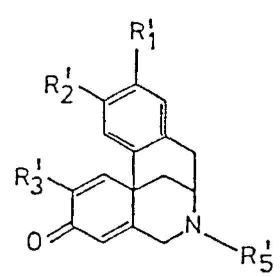

(III)

dans laquelle

R'$_1$ est un radical hydroxy ou alkoxy comprenant 1 à 3 atomes de carbone,

$R'_2$ est un atome d'hydrogène ou un radical alkoxy comprenant 1 à 3 atomes de carbone,

$R'_3$ est un radical alkoxy comprenant 1 à 3 atomes de carbone,

$R'_5$ est un atome d'hydrogène ou un radical alkyle comprenant 1 à 3 atomes de carbone ou un radical alkényle à chaîne ramifiée ou linéaire comprenant jusqu'à 5 atomes de carbone, un radical cycloalkylalkyle comprenant 4 à 7 atomes de carbone ou un radical phénéthyle dont le radical phényle pourrait être éventuellement substitué par 1 à 3 radicaux méthoxy ou éthoxy ou un groupe méthylènedioxy, et dans laquelle $R'_1$ combiné avec $R'_2$ présente éventuellement aussi un groupe méthylènedioxy aussi que les sels acceptables du point de vue pharmacologique.

2. Composition pharmaceutique avec une activité CNS, caractérisée par un composé au moins selon la revendication 1.

3. Procédé de production des composés selon formule générale I et de leurs dérivés N-substitués I

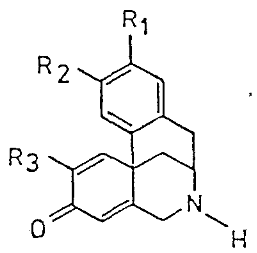

(I)

dans laquelle

$R_1$ est un radical hydroxy ou alkoxy comprenant 1 à 3 atomes de carbone,

$R_2$ est un atome d'hydrogène ou un radical alkoxy comprenant 1 à 3 atomes de carbone,

$R_3$ est un radical alkoxy comprenant 1 à 3 atomes de carbone, dans laquelle $R_1$ combiné avec $R_2$ présente éventuellement aussi un groupe méthylènedioxy, caractérisé par une cyclisation oxydante d'un dérivé 3-benzyle-1,2,3,4-tétrahydroisoquinoline de formule générale II

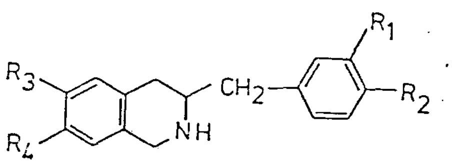

(II), (II)

dans laquelle les radicaux $R_1$, $R_2$ et $R_3$ sont tels que définis dans formule I et dans laquelle $R_4$ présente un radical alkoxy comprenant 1 à 3 atomes de carbone et dans laquelle l'atome d'hydrogène qui est attaché à l'atome nitrogène du cycle est éventuellement substitué par des radicaux qui ne sont pas changés par la réaction et qui ne peuvent pas inhiber le procédé de sa masse stérique qui est effectuée par des composés qui sont solubles dans la phase organique présente et qui présentent un potentiel d'oxydation suffisant et, le cas échéant, à N-substituer et convertir les composés de formule (I) en un sel acceptable du point de vue pharmacologique.

4. Procédé selon la revendication 3, caractérisé en ce que l'agent d'oxydation est le trifluorure de vanadyle en l'acide trifluoroacétique.

5. Procédé selon les revendications 3 à 4 caractérisé en ce que le trifluorure de vanadyle est employé avec un excès de 2,5 moles.

6. Procédé selon les revendications 3 à 5 caractérisé en ce que la température du procédé est entre – 15 °C et – 5 °C.